(19) Europäisches Patentamt
European Patent Office
Office européen des brevets

(11) EP 0 546 391 B1

(12) EUROPÄISCHE PATENTSCHRIFT

(45) Veröffentlichungstag und Bekanntmachung des Hinweises auf die Patenterteilung:
**08.05.1996 Patentblatt 1996/19**

(51) Int. Cl.$^6$: **C07C 213/02**, C07D 319/20, C07C 217/84

(21) Anmeldenummer: **92120190.1**

(22) Anmeldetag: **26.11.1992**

(54) **Verfahren zur Herstellung von Fluor enthaltenden Anilinen.**

Process for the preparation of fluorine containing anilines.

Procédé pour la préparation des anilines qui contiennent la fluorine.

(84) Benannte Vertragsstaaten:
**BE CH DE FR GB IT LI NL**

(30) Priorität: **09.12.1991 DE 4140536**

(43) Veröffentlichungstag der Anmeldung:
**16.06.1993 Patentblatt 1993/24**

(73) Patentinhaber: **BAYER AG**
**D-51368 Leverkusen (DE)**

(72) Erfinder: **Marhold, Albrecht, Dr.**
**W-5090 Leverkusen 1 (DE)**

(56) Entgegenhaltungen:
**EP-A- 0 315 862** **EP-A- 0 381 010**

- **BULLETIN DE LA SOCIETE CHIMIQUE DE FRANCE Nr. 6, 1986, PARIS FR Seiten 925 - 929 B. LANGLOIS, G. SOULA 'A safe and economical synthesis of 3-(trifluormethoxy aniline from 2-chlorophenol'**
- **JOURNAL OF ORGANIC CHEMISTRY Bd. 29, Nr. 1, 13. Januar 1964, EASTON US Seiten 1 - 11 W. A. SHEPPARD 'alpha-Fluorinated Ethers. I. Aryl Fluoroalkyl Ethers'**
- **WO 94/11349**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 546 391 B1

## Beschreibung

Die vorliegende Erfindung betrifft ein Verfahren zur Herstellung von in einer Seitenkette Fluor enthaltenden Anilinen aus den entsprechenden Chloraromaten.

Es ist bekannt, daß man 2-Trifluormethoxy-chlorbenzol mit Natriumamid zu 3-Trifluormethoxyanilin umsetzen kann (siehe EP-A2 0 140 783). Nachteilig ist dabei der Einsatz von teurem und schwierig zu handhabendem Natriumamid und die Unmöglichkeit, nach diesem Verfahren 2- und 4-Trifluormethoxy-aniline herzustellen.

Es ist ferner bekannt, daß man Trifluormethoxybenzol nitrieren und reduzieren und so Trifluormethoxyaniline erhalten kann (siehe Bull. Soc. Chim. France 1986, Nr. 6, S. 925). Nachteilig dabei ist, daß die Trifluormethoxyaniline stets in Form von ortho- und para-Isomerengemischen anfallen, da eine selektive Einführung der Nitrogruppe nicht möglich ist. Meta-trifluormethoxy-aniline sind auf diese Weise überhaupt nicht zuganglich. Diese Literaturstelle beschreibt auf Seite 926 auch die Herstellung von 3-Trifluormethoxy-4-chlor-anilin aus 2,6-Dichlor-trifluormethoxybenzol und Natriumamid. Dieses Verfahren ist aus den oben genannten Gründen nachteilig.

Schließlich ist bekannt, daß man 3-Nitrophenyl-fluorformiat mit Schwefeltetrafluorid umsetzen und das so erhältliche 3-Nitro-trifluormethoxybenzol zu 3-Trifluormethoxyanilin reduzieren kann (siehe J. Org. Chem. 29 (1), S. 1-11 (1964)). Der Einsatz des sehr toxischen und teuren Schwefeltetrafluorids ist allerdings sehr nachteilig, da es besonderen Aufwand erfordert, ebenso wie die Beseitigung des entstehenden Thionylfluorids.

Es wurde nun ein Verfahren zur Herstellung von in einer Seitenkette Fluor enthaltenden Anilinen der Formel (I) gefunden

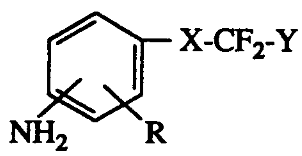

(I),

in der

R für Wasserstoff, Chlor, $C_1$-$C_4$-Alkyl oder $C_1$-$C_4$-Alkoxy steht,

X für Sauerstoff oder Schwefel steht und

Y für Fluor, $CF_3$ oder $CF_2Cl$ steht oder zusammen mit dem $CF_2$-X-Rest eine -O-$CF_2$-$CF_2$-O-Gruppe darstellt, deren beide Sauerstoffatome in o-Stellung zueinander an den aromatischen Ring gebunden sind,

das dadurch gekennzeichnet ist, daß man Chloraromaten der Formel (II)

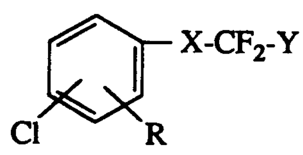

(II),

in der
R, X und Y die bei Formel (I) angegebene Bedeutung haben,
in Gegenwart eines Katalysators und gegebenenfalls Wasser bei 200 bis 280°C mit Ammoniak umsetzt.

Für das erfindungsgemäße Verfahren geeignete Chloraromaten der Formel (II) sind Handelsprodukte oder nach bekannten Verfahren herstellbar (siehe z.B. Houben-Weyl, Methoden der organischen Chemie, Band E4, S. 626-644(1983)).

Vorzugsweise werden in das erfindungsgemäße Verfahren Chloraromaten der Formel (II) eingesetzt, bei denen

R für Wasserstoff, Chlor, Methyl, Ethyl, Methoxy oder Ethoxy,

X für Sauerstoff und

Y für Fluor stehen.

Als Katalysator für das erfindungsgemäße Verfahren kommen beispielsweise Verbindungen von Eisen, Kobalt, Nikkel, Zink und Silber, in Mischungen mit Kupfer oder Kupferverbindungen, in Frage. Bevorzugt sind Halogenide von Nickel und Kupfer, gegebenenfalls im Gemisch mit Kupfer. Besonders bevorzugt sind Kupferoxid und Kupferhalogenide. Der Katalysator kann in das erfindungsgemäße Verfahren beispielsweise in einer Menge von 0,1 bis 20 Gew.-%, bezogen auf den eingesetzten Chloraromaten der Formel (II), eingesetzt werden. Vorzugsweise beträgt diese Menge 1 bis 15 Gew.-%.

Das erfindungsgemäße Verfahren kann in Anwesenheit oder in Abwesenheit von Wasser durchgeführt werden. Vorzugsweise wird es in Gegenwart von Wasser durchgeführt. Beispielsweise kann man in das erfindungsgemäße Verfahren Wasser in einer Menge von 20 bis 500 Gew.-%, bezogen auf den eingesetzten Chloraromaten der Formel (II), einsetzen. Vorzugsweise betragt diese Menge 50 bis 200 Gew.-%.

Der für das erfindungsgemäße Verfahren benötigte Ammoniak wird vorzugsweise in flüssiger Form zudosiert Man kann beispielsweise 40 bis 500 ml flüssigen Ammoniak pro 100 g des eingesetzten Chloraromaten der Formel (II) verwenden. Vorzugsweise beträgt diese Menge 50 bis 250 ml.

Das erfindungsgemäße Verfahren wird bei Temperaturen im Bereich 200 bis 280°C durchgeführt. Bevorzugt sind Temperaturen im Bereich 220 bis 260°C.

Im allgemeinen ist die erfindungsgemäße Umsetzung nach 5 bis 20 Stunden beendet.

Bei einer bevorzugten technischen Ausführungsform des erfindungsgemäßen Verfahrens wird wie folgt gearbeitet: In einem Autoklaven werden der Chloraromat der Formel (II), Wasser und der Katalysator vorgelegt und anschließend flüssiges Ammoniak zudosiert Dann wird unter Rühren für 5 bis 20 Stunden auf die gewünschte Reaktionstemperatur erhitzt. Danach wird der Autoklav auf Raumtemperatur abgekühlt, entspannt und überschüssiger Ammoniak zurückgewonnen. Schließlich wird die organische Phase mit Wasserdampf destilliert oder extrahiert und gegebenenfalls destilliert.

Das erfindungsgemäße Verfahren hat eine Reihe von Vorteilen. Es gestattet die Herstellung von in einer Seitenkette Fluor enthaltenden Anilinen der Formel (I) im allgemeinen mit guten Umsätzen und befreidigenden Ausbeuten. Wesentlich ist, daß beim erfindungsgemäßen Verfahren die Aminogruppe selektiv dort eintritt, wo vorher ein Chloratom gebunden war. Außerdem lassen sich beim Einsatz von Dichloraromaten der Formel (II) selektiv Monochloraniline der Formel (I) herstellen. Überraschenderweise treten keine Isomerengemische auf und die Y-$CF_2$-X-Gruppe, die in J. Am. Chem. Soc. 85 (2), S. 1314-1318 (1963) als Superhalogen bezeichnet wird, wird nicht gegen eine Aminogruppe ausgetauscht. Es konnte also nicht erwartet werden, daß das erfindungsgemäße Verfahren so gute Ergebnisse liefert.

Das erfindungsgemäße Verfahren eignet sich ganz besonders zur Herstellung von in einer Seitenkette Fluor enthaltenden Anilinen der Formel (I), bei denen

R für Wasserstoff, Chlor, Methyl, Ethyl, Methoxy oder Ethoxy,

X für Sauerstoff und

Y für Fluor oder $CF_3$ steht.

Insbesondere eignet sich das erfindungsgemäße Verfahren zur Herstellung der Trifluormethoxyaniline der Formel (III)

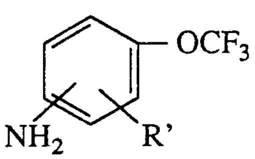

(III),

in der sich die $NH_2$-Gruppe in o- oder m-Stellung zur $CF_3O$-Gruppe befindet und in der R' für Chlor in o- oder m-Stellung zur $CF_3$-O-Gruppe oder für Methyl in p-Stellung zur $CF_3$-O-Gruppe steht.

Bevorzugte Trifluormethoxyaniline der Formel (III) sind 3-Trifluormethoxy-5-chlor-anilin, 2-Chlor-3-trifluormethoxy-anilin, 2-Trifluormethoxy-5-methyl-anilin und 2-Trifluormethoxy-3-chlor-anilin.

Die Herstellung der Trifluormethoxyaniline der Formel (III) ist weiter oben beschrieben. Die Trifluormethoxyaniline der Formel (III) können als Zwischenprodukte für Farbstoffe, Pflanzenschutzmittel und Pharmazeutika verwendet werden.

Beispiele

Allgemeines:

In einem Autoklaven wurden jeweils in den angegebenen Mengen ein Chloraromat der Formel (II), Wasser und Katalysator vorgelegt und dazu die jeweils angegebene Menge an flüssigem Ammoniak aufgedrückt. Unter Rühren wurde für die jeweils angegebene Zeit auf die jeweils angegebene Temperatur erhitzt. Danach wurde der Autoklav auf Raumtemperatur abgekühlt, entspannt, die organische Phase von der wäßrigen Phase getrennt und die organische Phase mit Wasserdampf destilliert. Aus dem Destillat wurde die organische Phase abgetrennt, ausgewogen und analysiert. Die im einzelnen durchgeführten Beispiele sind in der folgenden Tabelle zusammengefaßt.

Tabelle

| Beispiel Nr. | Chloraromat der Formel (II)*) R | Cl | X | Y | Menge (g) | Wassermenge (ml) | Ammoniakmenge (ml) | Katalysator und Menge (g) | | Temperatur (°C) | Reaktionszeit (h) | erhaltenes Anilin der Formel (I)*) R | $NH_2$ | X | Y | Umsatz (%) | Ausbeute (%) |
|---|---|---|---|---|---|---|---|---|---|---|---|---|---|---|---|---|---|
| 1 | H | 4 | 0 | F | 100 | 100 | 150 | CuCl | 10 | 250 | 6 | H | 4 | O | F | 86 | 78.8 |
| 2 | H | 4 | 0 | F | 50 | 50 | 50 | CuCl | 1 | 230 | 10 | H | 4 | O | F | 85 | 14,2 |
| 3 | H | 2 | 0 | F | 100 | 100 | 130 | CuCl | 15 | 250 | 15 | H | 2 | O | F | 93,8 | 72,3 |
| 4 | H | 2 | 0 | F | 100 | 100 | 150 | CuCl | 10 | 250 | 6 | H | 2 | O | F | 70 | 53,5 |
| 5 | H | 2 | 0 | F | 50 | 50 | 70 | CuBr | 5 | 240 | 10 | H | 2 | O | F | 71 | 28,8 |
| 6 | H | 3 | 0 | F | 100 | 100 | 130 | CuCl | 10 | 250 | 10 | H | 3 | O | F | 82 | 63 |
| 7 | H | 4 | S | F | 50 | 50 | 70 | CuCl | 3 | 250 | 7 | H | 4 | S | F | 90 | 88 |
| 8 | 3-Cl | 5 | O | F | 50 | 50 | 50 | CuCl | 5 | 240 | 10 | 3-Cl | 5 | O | F | 93 | 59 |
| 9 | 2-Cl | 3 | O | F | 100 | 100 | 120 | CuCl | 10 | 250 | 8 | 2-Cl | 3 | O | F | 52 | 18 |
| 10 | 4-$CH_3$ | 2 | O | F | 100 | 100 | 130 | CuCl | 10 | 250 | 15 | 4-$CH_3$ | 2 | O | F | 89 | 56 |
| 11 | 4-Cl | 3 | O | F | 50 | 50 | 60 | Cu/CuCl | 10 | 250 | 10 | 4-Cl | 3 | O | F | 92 | 54 |
| 12 | 2-Cl | 5 | O | F | 40 | 50 | 50 | CuCl | 5 | 250 | 10 | 2-Cl | 5 | O | F | 75,8 | 62 |
| 13 | H | 3-Cl | $-O(CF_2)_2O-$ 5,6 | | 14 | 30 | 30 | CuCl | 1 | 250 | 8 | H | 3 | $-O(CF_2)_2O-$ 5,6 | | 100 | 51 |
| 14 | 2-Cl | 4 | O | F | 200 | 200 | 200 | CuCl | 15 | 230 | 7 | 2-Cl | 4 | O | F | 53 | 87,5 |
| 15 | 6-Cl | 2 | O | F | 100 | 100 | 100 | CuCl | 10 | 240 | 10 | 6-Cl | 2 | O | F | 77 | 72,4 |
| 16 | H | 4 | O | $CF_3$ | 100 | 100 | 120 | CuCl | 15 | 250 | 10 | H | 2 | O | F | 84 | 77,5 |

*) Zahlen bedeuten die Stellung relativ zur Y-$CF_2$-X-Gruppe, O bedeutet Sauerstoff, S bedeutet Schwefel, F bedeutet Fluor

Physikalische Daten einiger hergestellten Verbindungen der Formel (I).

3-Chlor-5-amino-trifluormethoxybenzol (Beispiel 8):

Siedepunkt: 94 bis 95°C bei 12 mbar

Brechungsindex $n_D^{20}$ : 1,4940

2-Chlor-3-amino-trifluormethoxybenzol (Beispiel 9):

Siedepunkt: 102 bis 103°C bei 14 mbar

Brechungsindex $n_D^{20}$ : 1,4982

2-Amino-4-methyl-trifluormethoxybenzol(Beispiel 10):

Siedepunkt: 40 bis 41°C bei 10 mbar

Brechungsindex $n_D^{20}$ : 1,4435

2-Amino-6-chlor-trifluormethoxybenzol (Beispiel 15):

Siedepunkt: 92 bis 94°C bei 15 mbar

Brechungsindex $n_D^{20}$ : 1,5020

## Patentansprüche

1. Verfahren zur Herstellung von in einer Seitenkette Fluor enthaltenden Anilinen der Formel (I)

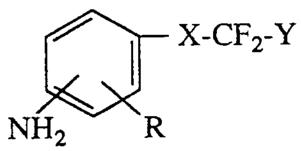

(I),

in der

R für Wasserstoff, Chlor, $C_1$-$C_4$-Alkyl oder $C_1$-$C_4$-Alkoxy steht,

X für Sauerstoff oder Schwefel steht und

Y für Fluor, $CF_3$ oder $CF_2Cl$ steht oder zusammen mit dem $CF_2$-X-Rest eine -O-$CF_2$-$CF_2$-O-Gruppe darstellt, deren beide Sauerstoffatome in o-Stellung zueinander an den aromatischen Ring gebunden sind,

das dadurch gekennzeichnet ist, daß man Chloraromaten der Formel (II)

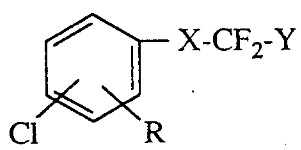

(II),

in der
R, X und Y die bei Formel (I) angegebene Bedeutung haben,
in Gegenwart eines Katalysators und gegebenenfalls Wasser bei 200 bis 280°C mit Ammoniak umsetzt.

2. Verfahren nach Anspruch 1, dadurch gekennzeichnet, daß man als Katalysator Verbindungen von Eisen, Kobalt, Nickel, Zink und Silber in Mischungen mit Kupfer oder Kupferverbindungen einsetzt.

3. Verfahren nach Anspruch 1, dadurch gekennzeichnet, daß man als Katalysator Kupferoxid oder Kupferhalogenide einsetzt.

4. Verfahren nach Ansprüchen 1 bis 3, dadurch gekennzeichnet, daß man es in Gegenwart von 20 bis 500 Gew.-% Wasser, bezogen auf den eingesetzten Chloraromaten der Formel (II), durchführt.

5. Verfahren nach Ansprüchen 1 bis 4, dadurch gekennzeichnet, daß man pro 100 g eingesetzten Chloraromaten der Formel (II) 40 bis 500 ml flüssigen Ammoniak einsetzt.

## Claims

1. Process for the preparation of anilines of the formula (I) which contain fluorine in a side chain,

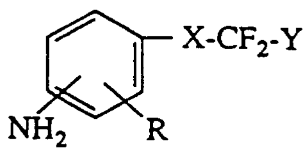

(I)

in which

R represents hydrogen, chlorine, $C_1$-$C_4$-alkyl or $C_1$-$C_4$-alkoxy,

X represents oxygen or sulphur and

Y represents fluorine, $CF_3$ or $CF_2Cl$ or, together with the $CF_2$-X radical, represents an -O-$CF_2$-$CF_2$-O-group whose two oxygen atoms are bonded in the o-position to each other in the aromatic ring,

characterised in that chlorinated aromatic compounds of the formula (II)

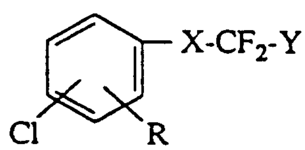

(II)

in which
R, X and Y have the meaning given for the formula (I),
are reacted with ammonia in the presence of a catalyst and optionally water at 200 to 280°C.

2. Process according to Claim 1, characterised in that the catalysts used are compounds of iron, cobalt, nickel, zinc and silver mixed with copper or copper compounds.

3. Process according to Claim 1, characterised in that the catalysts used are copper oxide or copper halides.

4. Process according to Claims 1 to 3, characterised in that it is performed in the presence of 20 to 500% by weight of water, relative to the chlorinated aromatic compound of the formula (II) which is used.

5. Process according to Claims 1 to 4, characterised in that 40 to 500 ml of liquid ammonia are used per 100 g of the chlorinated aromatic compound of the formula (II) which is used.

## Revendications

1. Procédé pour la préparation d'anilines contenant du fluor dans une chaîne latérale, répondant à la formule (I)

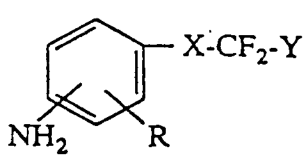

(I),

dans laquelle

R représente un atome d'hydrogène, un atome de chlore, un groupe alkyle en $C_1$-$C_4$ ou un groupe alcoxy en $C_1$-$C_4$,

X représente un atome d'oxygène ou un atome de soufre, et

Y représente un atome de fluor, $CF_3$ ou $CF_2Cl$ ou représente, conjointement avec le radical $CF_2$-X, un groupe -O-$CF_2$-$CF_2$-O- dont les deux atomes d'oxygène en position o l'un par rapport à l'autre sont liés au noyau aromatique,

qui est caractérisé par le fait qu'on fait réagir des composés aromatiques chlorés répondant à la formule (II)

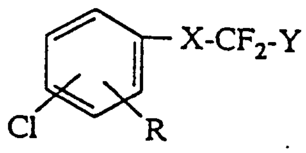

(II),

dans laquelle
R, X et Y ont la signification indiquée à la formule (I),
en présence d'un catalyseur et éventuellement d'eau, à une température de 200 à 280°C, avec de l'ammoniac.

2. Procédé selon la revendication 1, caractérisé en ce qu'on met en oeuvre, comme catalyseur, des composés du fer, du cobalt, du nickel, du zinc et de l'argent dans des mélanges avec du cuivre ou des composés contenant du cuivre.

3. Procédé selon la revendication 1, caractérisé en ce que, comme catalyseur, on met en oeuvre de l'oxyde de cuivre ou des halogénures de cuivre.

4. Procédé selon les revendications 1 à 3, caractérisé en ce qu'on l'effectue en présence de 20 à 500% en poids d'eau rapportés aux composés aromatiques chlorés de formule (II) mis en oeuvre.

5. Procédé selon les revendications 1 à 4, caractérisé en ce que, pour 100 g de composés aromatiques chlorés de formule (II) mis en oeuvre, on met en oeuvre de 40 à 500 ml d'ammoniac liquéfié.